(19) **Europäisches Patentamt European Patent Office Office européen des brevets**

(11) **EP 4 749 312 A1**

(12) **EUROPEAN PATENT APPLICATION**

(43) Date of publication:
**27.05.2026 Bulletin 2026/22**

(21) Application number: **24214298.2**

(22) Date of filing: **20.11.2024**

(51) International Patent Classification (IPC):
**G01R 33/12** *(2006.01)* **A61B 5/0515** *(2021.01)*

(52) Cooperative Patent Classification (CPC):
**G01R 33/1276; A61B 5/0515**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Bruker BioSpin GmbH & Co. KG 76275 Ettlingen (DE)**

(72) Inventors:
• **ILBEY, Serhat 76337 Waldbronn (DE)**

• **FRANKE, Jochen 76199 Karlsruhe (DE)**

(74) Representative: **Kohler Schmid Möbus Patentanwälte Partnerschaftsgesellschaft mbB Gropiusplatz 10 70563 Stuttgart (DE)**

Remarks:
Amended claims in accordance with Rule 137(2) EPC.

(54) **METHOD FOR CALCULATING A MULTI-DIMENSIONAL IMAGE-DOMAIN SYSTEM MATRIX FOR MPI MEASUREMENTS USING A MULTI-DIMENSIONAL SINOGRAM-BASED SYSTEM MATRIX**

(57)    The invention concerns a method for generating a multi-dimensional image-domain system matrix $A$ using an MPI-sequence with a field free line, using a multi-dimensional sinogram-based system matrix

$$A_{Sinogram}(t,r,z) \in \mathbb{R}^{[T,N_r,N_z]},$$

comprising the following steps:
• for each time point $t_c$, $c \in [1,T]$:
◦    get a time slice matrix

$$A_c = A_{Sinogram}(t=t_c,r,z) \in \mathbb{R}^{[N_r,N_z]},$$

◦ replicate the time slice matrix $A_c$ $N_r$ - 1 times and copying these replications along a newly added 2nd offset-dimension, resulting in a time volume matrix $A'_c \in \mathbb{R}^{[N_r,N_r,N_z]}$,

◦ rotate the time volume matrix $A'_c$ by the FFL angle $\theta_c = \theta$ ($t = t_c$) at time point $t = t_c$ in its 1st and 2nd offset-dimensions, resulting in a rotated matrix $A_c^{\theta_c}$

• concatenate the rotated matrices $A_c^{\theta_c}$ of all time points along a time-dimension and vectorize the spatial dimensions ($x,y,z$), resulting in a constructed system matrix

$$cSM \in \mathbb{R}^{[T,(N_r \times N_r \times N_z)]},$$

• perform a Fourier transformation on the constructed system matrix $cSM$ on its time-dimension resulting in the multi-dimensional image-domain system matrix $A$.

With the inventive method the total calibration time can be lowered.

EP 4 749 312 A1

Fig. 1

**EP 4 749 312 A1**

## Description

Field of invention

The invention concerns a method for calculating a multi-dimensional image-domain system matrix acquired from calibration measurements performed by an MPI Scanner using an MPI-sequence with a field free line FFL with FFL angles, FFL offsets r and FFL velocities.

Background of the invention

In magnetic particle imaging (MPI) measurements, a spatially dependent magnetic field with a field-free region (e.g. a field-free point FFP or a field-free line FFL) is applied (selection field). FFL topology is in particular preferred for multidimensional imaging because it provides higher sensitivity than FFP topology, as all SPIONs across the entire FFL contribute to the signal [Weizenecker2008]. By applying a magnetic shift/focus field, the field-free region is moved through an examination volume (field of view FOV) along a trajectory with the aid of a measurement sequence in a drive-field region. The signal responses of magnetic particles, preferably superparamagnetic iron oxide particles (SPION), are measured as MPI signal data. The measured signal data are displayed as a sum signal of all excited particles. These are measured in the time domain and transformed into the frequency domain by a Fourier transform. A reconstruction is performed to produce the MPI image, e.g. using a system-function-based MPI image reconstruction.

In case of a system-function-based MPI image reconstruction, knowledge of a spatially encoded system response (frequency response), a so-called system function, is used, the latter describing the relationship between the measurement signal (MPI signal data) and, for example, the particle distribution of a certain particle class (mapping the particle concentration on a measured frequency response). As a rule, the system function is available as a "system matrix", i.e. as discretized system function. The system matrix is provided for a system matrix region that comprises the part of the image domain where MPI image data are to be reconstructed. The system matrix describes the signal response of a calibration sample at various spatial positions. The system matrix is determined independently of the actual object measurement (e.g., by calibration measurement, by simulation, by hybrid approaches).

For the conventional system matrix acquisition, a system calibration sequence is performed repeating the MPI sequence to be performed and a calibration sample is placed at each voxel (3D pixel) position within the field of view (FOV). These calibration measurements are then used to construct the system matrix, designated with $A^{M \times N}$, where $M$ represents the number of the frequency components used, and $N$ is the number of voxels. Finally, the image is reconstructed solving the ill-conditioned linear system of equations

$$A\rho = b$$

using iterative optimization methods, such as regularized Kaczmarz and FISTA, with the forward model

$$\underset{\rho}{\mathrm{argmin}} \; \|A\rho - b\|_2^2 + \lambda \Phi(\rho)$$

where $\rho$ is the image to be reconstructed, $b$ is the measurement vector, $\Phi(\cdot)$ is the regularization operator (for example, $\ell_1$- and $\ell_2$-norm of the image: $\|\rho\|_1$ and $\|\rho\|_2$, respectively), and $\lambda$ is the regularization constant.

However, a fully-measurement-based calibration process can be impractical due to limited signal-to-noise ratio (SNR) and the need for a highly precise MPI-compatible 3D-axis-robot, which operates at limited positioning speed. As a result, acquiring a high-resolution system matrix can take several days depending on measurement time and the number of averages [Weizenecker2009]

A classical system matrix calibration is disclosed in [US2015221103]. This calibration method applies compressed sensing steps with a transformation matrix, which sparsifies the image reconstruction matrix, wherein the transformation matrix comprises analytic transformations.

[US20170020407] describes an MPI method to determine calibration and measurement volumes, wherein the calibration volume is larger than the measurement volume and the overall measurement volume is arranged within the calibration volume ("overscanning").

[Kaethner] discloses a method for acquiring MPI data based on the axial elongation of a 2D FFP-trajectory. It is shown that such an elongation can be used as a data acquisition path to significantly increase the acquisition speed, with negligible loss of spatial resolution. However, as a result of the elongation, the 3D measurement volume is not sampled symmetrically and / or homogeneously which may lead to distortions and information loss in the reconstructed images.

[Von Gladiss] discloses the acquisition of a hybrid system matrix with a magnetic particle spectrometer. In contrast to changing the nanoparticle sample position, the nanoparticle sample stays at the same spatial position inside the sample chamber. Different spatial positions are emulated inside the magnetic particle spectrometer by changing the magnetic field that is applied to the particles. When acquiring a hybrid system matrix with a magnetic particle spectrometer the whole sample chamber represents one infinitesimal volume of the FOV in an MPI scanning device. Consequently, the calibration sample size is not constrained to the dimensions of a voxel of targeted image resolution, allowing for the use of a calibration sample as large as the sample chamber itself, provided the magnetic fields are homogeneous throughout, enabling high-SNR calibration measurements. The application of homogeneous offset fields generates the field sequence of this volume for all the particles inside the sample chamber. However, the magnetization response of SPIONs can be influenced by environmental factors such as temperature, and viscosity. Additionally, the non-deterministic field drifts and non-linearities present in the MPI scanner etc. may not always be accurately emulated with a magnetic particle spectrometer and need to be compensated (e.g. transfer function compensation, background correction, sensitivity profile correction,...).

[CN115797493] discloses a MPI sampling based on a FFL using an one-dimensional system matrix, where each projection angle (FFL angle) of the 2D imaging object is acquired separately in a step-wise manner. However, this method does not always work accurately and can only be used for a specific type of sequence.

[US009651637] discloses a method for filtering out interferences from a received signal by applying signal-processing techniques like spectral cleaning.

[Halkola] discloses a method for generating a system matrix using an FFP. Instead of moving the nanoparticle sample position by a robot, magnetic focus fields are used for estimating the system function. This approach allows calibration measurements to be performed either using an external magnetic particle spectrometer (MPS) [Hybrid] or the MPI system itself without the need for additional hardware, thereby increasing the measurement duty cycle up to 100% by eliminating positioning time of the robot. Nevertheless, the full calibration procedure can still take several hours, during which the MPI system remains unavailable for imaging. Further, this method is not applicable for calibration measurements using FFL.

When using a field-free-line topology, the system function can be derived from a reduced dataset by leveraging the shift and rotation invariance of the MPI system [Mohn]. For instance, the MPI signals at various FFL angles can be synthesized from a single-angle calibration measurement [Li]. However, to accurately obtain the field-based system matrix applying constant (i.e., dc) focus field offsets, the FFL angle must remain fixed between repetitions.

Object of the invention

It is an object of the invention to provide a method for lowering the total calibration time for generating a system matrix compared to conventional calibration and/or reducing mechanical effort required to position the source during calibration measurements.

Summary of the invention

This object is achieved by a method for generating a multi-dimensional image-domain system matrix according to claim 1.

According to the invention, a multi-dimensional sinogram-based system matrix $A_{Sinogram}(t, r, z) \in \mathbb{R}^{[T, N_r, N_z]}$, is used to generate the multi-dimensional image-domain system matrix $A$, where

$r$ = offset in the offset-dimension of the sinogram

$\theta$ = angle in the angle-dimension of the sinogram

$t$ = time in the time-dimension of the multi-dimensional sinogram-based system matrix $A_{Sinogram}$

$N_r$ = number of offsets

$N_z$ = number of voxels of a region of interest in a $z$-direction

$T$ = number of time points of the time dimension (number of calibration data points during an MPI imaging sequence)

The sinogram-based system matrix is acquired from calibration measurements using a field free line FFL with FFL angles, FFL offsets, and FFL velocities, the sinogram having an offset-dimension, an angle-dimension, and a $z$-dimension. The FFL is generated by means of selection fields and shifted by means of focus fields of an MPI scanner.

The inventive method comprises the following steps:

For each time point $t_c$, $c \in [1, T]$

- get a time slice matrix $A_c = A_{Sinogram}(t = t_c, r, z) \in \mathbb{R}^{[N_r, N_z]}$, which comprises calibration data of the multi-dimensional sinogram-based system matrix $A_{sinogram}(t,r,z)$ at time point $t = t_c$, the time slice matrix having a 1st offset-dimension $r$ and a z-dimension $z$,

- replicate the time slice matrix $A_c$ for each FFL-offset $r$, resulting in $N_r$ identical time slice matrices $A_c$, and copying these replications along a newly added 2nd offset-dimension, resulting in a time volume matrix $A_c' \in \mathbb{R}^{[N_r, N_r, N_z]}$, and

- rotate the time volume matrix $A_c'$ by the FFL angle $\theta_c = \theta(t = t_c)$ of the respective field free line FFL at time point $t = t_c$ in its 1st and 2nd offset-dimensions, resulting in a rotated matrix $A_c^{\theta_c}$, where the dimensions of $A_c^{\theta_c}$ are associated with the spatial dimensions $(x,y,z)$.

The rotated matrices $A_c^{\theta_c}$ of all time points are concatenated along a time-dimension and the spatial dimensions $(x,y,z)$ are vectorized, resulting in a constructed system matrix $cSM \in \mathbb{R}^{[T, (N_r \times N_r \times N_z)]}$.

Finally, the multi-dimensional image-domain system matrix $A$ is obtained after performing a Fourier transformation on the constructed system matrix $cSM$ in its time-dimension.

The steps outlined above assume that the FFL rotation and FFL translation occur in the *xy*-plane, with a drive field (DF) defined along the *z*-axis. These steps should be modified as needed to accommodate different hardware configurations.

The replication of the time slice matrix is performed because it is assumed that the signals received from the spatial positions along the FFL are identical. This assumption best holds if the FFL is straight with zero field gradient along and at least as long as the scanner bore diameter.

At a given time point, the FFL has a specified FFL-angle. For rotating the time volume matrix, a rotation operator is applied to time volume matrix. Each slice z (slice of the time volume matrix having the same z-value) of the time volume matrix is rotated by the FFL-angle $\theta_c$ of the FFL at the respective time point. By rotating of the time volume matrix, the data of the calibration measurements is converted from sinogram domain to image domain using the FFL angle $\theta_c$ of the trajectory at the given time point.

For concatenating the rotated matrices, it is preferred to first create a matrix of zeros $A_{traj} \in \mathbb{R}^{[T, N_r, N_r, N_z]}$.

Then, the rotated matrices $A_c^{\theta_c}$ are stacked into the matrix of zeros $A_{traj}$ along the first (time) dimension. By vectorizing the spatial dimensions of $A_{traj}$, the constructed system matrix cSM is obtained.

The steps described above can be summarized as follows:

$$cSM = V \left\{ \begin{bmatrix} A_1^{\theta_1} \\ A_2^{\theta_2} \\ \vdots \\ A_T^{\theta_T} \end{bmatrix} \right\} = V \left\{ \begin{bmatrix} \mathcal{R}_{\theta_1}\{\Psi\{A_1\}\} \\ \mathcal{R}_{\theta_2}\{\Psi\{A_2\}\} \\ \vdots \\ \mathcal{R}_{\theta_T}\{\Psi\{A_T\}\} \end{bmatrix} \right\}$$

where $\Psi$ is the operator replicating the 1st offset dimension of $A_c$ of size $N_r \times N_z$, resulting in a $N_r \times N_r \times N_z$ matrix, and $V\{\cdot\}$ is the operator, which vectorizes the spatial dimensions. By concatenating the rotated matrices, the conventional image-domain (robot-like) calibration data is constructed. In other words, in ideal conditions, i.e. where SNR is ultra-high, FFL is infinitely long, perfectly straight, and the fields of the MPI system are shift- and rotation invariant, the constructed system matrix $cSM$ comprises data identical to the data that is acquired with a conventional robot-based calibration, where a calibration source is moved within the entire 3D field of view.

The multidimensional image-domain system matrix which should be used for reconstruction of MPI data is obtained by applying a Fourier transform to the constructed system matrix:

$$A = \mathcal{F}\{cSM\}$$

where $\mathcal{F}$ is the Fourier transform operator along the time-dimension, and the resulting system matrix $A$ is a $K \times N$ matrix,

where K is the number of frequency components, and $N = N_{xy} \times N_{xy} \times N_z$ is the total number of voxels of the calibration volume, assuming $N_r = N_{xy}$.

In case there is no shift and rotation invariance, correction fields have to be determined.

The calibration signals of the calibration measurements are detected by one or more receiver coils. If multiple receiver coils are used, **A** must be calculated for each coil separately.

The multi-dimensional sinogram-based system matrix $A_{Sinogram}$ can be obtained in different ways:

In a **first variant (trajectory offset variant),** the multi-dimensional sinogram-based system matrix $\boldsymbol{A_{Sinogram}}(t, r, z) \in \mathbb{R}^{[T, N_r, N_z]}$ is generated according to the following method, wherein the offset $\Delta r$ is an FFL trajectory offset, i.e. a shift of the complete trajectory:

Prior to calibration measurements, the following is determined:

- target positions for a calibration source,

- a calibration volume in sinogram domain from which calibration data is to be acquired, the calibration volume comprising the region of interest and optionally an overscanning region (The region of interest is the region to be imaged; the overscanning region is the area of the calibration volume that is not to be imaged, but is used for calibration measurements.)

- MPI imaging sequence with a trajectory comprising sequence parameters, the sequence parameters including FFL angular frequency, FFL translational frequency (rate of the change of the translational velocity during a trajectory run), and MPI imaging sequence duration,

- Tracer and/or environmental parameters,

- FFL-trajectory-offsets $\Delta r$, by which the positions of the field free line during the trajectory are to be shifted within the calibration volume, perpendicular to the respective orientation of the field free line,

The calibration measurements are performed by running the MPI imaging sequence/trajectory at least once during in each calibration measurement using an MPI scanner, wherein for each combination of FFL-trajectory-offset $\Delta r$ and target position **z** a calibration measurement is performed,

The multi-dimensional sinogram-based system matrix $\boldsymbol{A_{Sinogram}}$ is determined from the calibration data.

The calibration data are acquired by applying a drive field to a calibration source (DF-excitation) and detecting the signal response.

The determined multi-dimensional sinogram-based system matrix is valid for specific tracer and/or environmental parameters. In case the tracer and/or environmental parameters change a new multi-dimensional sinogram-based system matrix has to be determined.

Instead of a conventional calibration, which is done by repeating the MPI sequence (e.g., with the sinusoidal trajectory) by positioning a point-like source object at every point in the imaging volume, the MPI sequence is repeated for varying FFL trajectory offsets in sinogram domain. I.e. the FFR trajectory offset is applied to the entire trajectory, i.e. each position of the FFL during the trajectory is shifted perpendicular to the orientation of the FFL at the specific position. The FFL velocities and FFL angles vary dynamically. After each calibration measurement (comprising at least one trajectory run) the FFL trajectory offset is changed. In contrast to conventional calibration, MPI sequence repetitions preferably comprise stepped target positions z, and FFL trajectory offsets that are not along the FFL-directions, since it is assumed that the signals received from the spatial positions along the FFL are identical. Thus, the number of calibration measurements can be significantly reduced using this dimensionality reduction.

In some variants the number of offset $N_r$ can be chosen smaller than the number of calibration voxels in the $xy$-plane, $N_r \leq N_{xy}$ (if overscanning is ignored).

In some variants the number of offset $N_r$ can be chosen larger than the number of calibration voxels in the $xy$-plane, $N_r > N_{xy}$.

Preferably, the target positions of the calibration source only vary in drive field direction. Drive field(s) are orthogonal to the focus fields driving the FFL angular frequency and FFL translational frequency. For example, FFL rotation and FFL translation are defined in the $xy$-plane and the drive field is defined in the $z$-dimension. In a special embodiment, there can be multiple drive field and focus field directions and drive fields and focus fields can be in the same dimension (superposition).

Since the invention is focused on system matrices for reconstruction of MPI data acquired using FFL, the trajectory is defined in the sinogram domain (r, $\theta$). Accordingly, the calibration volume is defined in sinogram domain.

During each calibration measurement, the entire trajectory is run at least once, wherein for different calibration measurements the trajectory is run with different offsets. Averaging can be applied. If no averaging is applied, one calibration measurement comprises one full trajectory. If averaging is applied, one calibration measurement comprises a plurality of trajectory runs. Each calibration measurement results in one vector of data points.

I.e. without averaging, the trajectory of the MPI sequence is run through $N_z$ times for each FFL-trajectory-offset $\Delta r$ and $N_r$ times for each target position z for the calibration source; in case of averaging, a multiple of the afore mentioned numbers of trajectory runs is performed.

For determining the multi-dimensional sinogram-based system matrix from the calibration data, calibration data is acquired for various combinations of FFL-offset and source positions, each calibration data having T time points. The resulting data T × Nr × Nz form the multi-dimensional sinogram-based system matrix $\boldsymbol{A}_{Sinogram}$.

Since the acquired calibration data originates from MPI hardware components such as power amplifiers, filters, etc., interferences with MPI receive coils can occur. In order to remove such interferences, signal-processing techniques like spectral cleaning can be applied to all calibration data and imaging data.

Preferably performing the calibration measurements comprises the following steps:

i. The calibration source is positioned within the calibration volume at a first target position,

ii. Calibration data is acquired with the calibration source being positioned at a first target position are performed, wherein the trajectory of the MPI sequence is run through until all FFL-trajectory-offsets $\Delta r$ have been set, wherein the FFL-trajectory-offset $\Delta r$ is changed after the trajectory is run through.

iii. The calibration source is positioned within the region of interest at a further target position that is shifted relative to the previous target position within the calibration volume in at least one direction, preferably along a drive field direction.

iv. The trajectory of the MPI sequence is run through until all FFL-trajectory-offsets $\Delta r$ have been set, wherein the FFL-trajectory-offset $\Delta r$ is changed after the trajectory is run through with the calibration source being positioned at the further target position,

v. Steps iii) - iv) are repeated until measurements for all target positions of the calibration source are performed.

In order to remove background noise, background measurements can be performed, preferably prior to positioning the calibration source at the target positions in step i and step iii, without the calibration source being in the calibration volume and/or with the calibration source being in a magnetically saturated state.

In a **second variant (MPS variant),** the multi-dimensional sinogram-based system matrix $\boldsymbol{A}_{Sinogram}(t, r, z) \in \mathbb{R}^{[T, N_r, N_z]}$ is generated according to the following method using a Magnetic Particle Spectrometer for emulating a calibration volume the method comprising:

Prior to calibration measurements, determine:

- calibration volume in sinogram domain, from which calibration data is to be acquired, the calibration volume comprising the region of interest,

- MPI scanner parameters of the MPI scanner including drive field frequency, drive field amplitude, and magnetic field gradient,

- MPI imaging sequence with a trajectory comprising sequence parameters, the sequence parameters including FFL angular frequency, FFL translational frequency, and MPI imaging sequence duration,

- FFL-trajectory-offsets $\Delta r$, by which the MPI imaging sequence is to be shifted within the calibration volume,

- spatial positions z of the calibration volume,

- tracer and/or environmental parameters,

- for each combination of FFL-trajectory-offset $\Delta r$, and spatial position z: time dependent magnetic fields B(t, $\Delta r$, z), wherein each magnetic field B(t, $\Delta r$, z) corresponds to the magnetic field at generated by the MPI scanner during a trajectory run with a specific FFL trajectory offset $\Delta r$ at a specific spatial position z,

Calibration measurements are performed by emulating the calibration volume by applying the determined magnetic fields to a calibration source, wherein for each combination of FFL-trajectory-offset $\Delta r$ and spatial position $z$ a calibration measurement is performed, during which the corresponding magnetic field (t, $\Delta r$, $z$) is applied at least once, and calibration data are acquired,

The multi-dimensional sinogram-based system matrix $A_{Sinogram}$ is determined from the calibration data.

The calibration data are acquired by applying a drive field to a calibration source (DF-excitation) and detecting the signal response.

The determined multi-dimensional sinogram-based system matrix is valid for specific tracer and/or environmental parameters. In case the tracer and/or environmental parameters change a new multi-dimensional sinogram-based system matrix has to be determined.

In contrast to the aforementioned variant, the calibration volume (i.e. the magnetic fields at different positions within the FOV of the MPI scanner) is emulated by an external Magnetic Particle Spectroscope. To emulate a calibration volume means emulating the fields generated by an MPI scanner during an MPI sequence for each repetition ($N_r \times N_z$).

The calibration source is positioned in a sample chamber of the MPS. By applying the emulated fields the calibration source experiences the same magnetic field as it would be positioned at a specific z-position in an MPI scanner while the trajectory is run with a specific trajectory offset (emulated calibration volume).

In a third **variant (generic variant)** the multi-dimensional sinogram-based system matrix $A_{Sinogram}(t,r,z) \in \mathbb{R}^{[T,N_r,N_z]}$ is generated according to the following method, wherein the offset $r$ is an FFL-offset $r$:

- perform generic MPI calibration measurements by applying at least one drive field cycle of a drive field in each calibration measurement using an MPI scanner, wherein during each generic MPI calibration measurement data is acquired for a specific combination of values of target position for a calibration source, FFL offset, and of predetermined parameters, comprising FFL angle and FFL velocities,

- determine a generic system matrix $A_{generic}$, where $A_{generic}$ comprises calibration data acquired from the generic MPI calibration measurements, and

From the generic system matrix $A_{generic}$, trajectory-specific calibration data is selected according to the following method:

- for each drive field cycle of an MPI measurement

    ○ determine the FFL angle, FFL offset, and FFL velocity,

    ○ pick the calibration data from the generic system matrix $A_{generic}$ of that calibration measurement that best matches to the combination of values of the predetermined parameters of the respective drive field cycle, and

    ○ concatenate all picked calibration data along the time dimension, resulting in the multi-dimensional sinogram-based system matrix $A_{Sinogram}$.

Instead of trajectory-specific calibration measurements, a generic set of measurements (i.e. independent of any trajectory for a multitude of parameter value combinations) are performed so that the calibration data can be used for a multitude, in particular for all possible MPI sequences. I.e. a generic system matrix is generated. The dimensions of the generic system matrix correspond to the parameters, which are varied for the different MPI calibration measurements.

Prior to the generic MPI calibration measurements, the following is determined:

    ○ target positions for a calibration source,

    ○ parameters, wherein the parameters include sequence parameters such as FFL velocities of a field free line FFL of a magnetic selection field, and may comprise further parameters

    ○ FFL-offsets, by which the field free line FFL is to be shifted within the calibration volume,

In variant 2 and variant 3, the number of offset $N_r$ is typically equal to the number of calibration voxels in the *xy*-plane, $N_r = N_{xy}$. In case of overscanning and / or oversampling, $N_r > N_{xy}$.

In a special variant, the parameters are discretized, thereby generating intervals of discretized parameter values for each parameter. The generic system matrix is then determined by determining individual acquisition system matrices

$A_c$, wherein each individual acquisition system matrix $A_c$ combines calibration data derived from different calibration measurements that are assigned to the same parameter value combination but different FFL-offsets, and combining individual acquisition system matrices $A_c$ of different parameter value combinations. The MPI-sequence is divided into trajectory-sections, whereby the duration of each trajectory-section corresponds to the duration of a drive field cycle, each trajectory-section being associated with an actual parameter value combination, wherein during each trajectory-section a data-subset of the MPI-data is acquired. For each trajectory-section, the best matching individual acquisition system matrix $A_c$ is picked from the multi-dimensional generic system matrix $A_{generic}$, wherein the best matching individual acquisition system matrix $A_c$ is the one with the parameter value combination that best matches the actual parameter value combination of the respective trajectory-section, $c^{+\mathbb{Z}} \in [1, C]$ is the trajectory-section number and $C$ is the total number of trajectory-sections. In this variant, calibration measurements are not performed by running trajectories, but by shifting the FFL to different offset-position, wherein for each offset position calibration measurements for a multitude of parameter value combinations are performed.

Preferably, the determined parameters comprise at least one of the following: particle type of used tracer of the calibration source, temperature of the calibration source, viscosity of the calibration source, velocity of the calibration source per acquisition window, DF frequency, DF amplitude, magnetic field gradient of an MPI system used for the MPI measurements, duration of the acquisition window, position of optional hardware inserts, shape of the field-free line. Although the consideration of additional parameters increases the total acquisition time and memory requirements, it enables the use of the same multidimensional system matrix for any arbitrary trajectory in a multitude of framework conditions. In this way, the MPI system user does not need to do very tedious calibration measurements repeatedly for each further parameter.

Alternatively, to the variant described so far, the multi-dimensional sinogram-based system matrix can be synthesized using model-based and/or hybrid MPI calibration techniques.

In a preferred variant, for setting a specific FFL-trajectory-offset the MPI sequence (first variant), or for setting a specific FFL offset (third variant) the field free line, is shifted in the offset-dimension of the sinogram by applying additional focus fields. The FFL-trajectory-offset or the FFL offset respectively can exceed half of the field of view to enable overscanning.

In case of limitations generating FFL-offsets due to, for example, limited peak power of power amplifiers, bore size, and overheating of hardware etc., the data can only be obtained for a limited range of field offsets, wherein "field offset" can be composed of the FFL-offset, the FFL-trajectory offset a z-offset or a combination thereof. The multi-dimensional sinogram-based system matrix $\boldsymbol{A_{Sinogram}}$ becomes, therefore, clipped in at least one of the spatial dimensions ($r,z$) of the multi-dimensional sinogram-based system matrix $\boldsymbol{A_{Sinogram}}$, such that the multi-dimensional sinogram-based system matrix $\boldsymbol{A_{Sinogram}}$, only comprises calibration data from field offsets lower than a threshold $r_{lim}, z_{lim}$.

If calibration data cannot be acquired for positions where $r > r_{lim}$ and/or $z > z_{lim}$, these data gaps should be estimated using interpolation, extrapolation, or advanced techniques such as artificial intelligence, where $r_{lim}$ = Maximum FFL-offset in the *xy*-plane, for example the MPI system bore radius and $z_{lim}$ = Maximum FFL-offset or position offset of the calibration source along the *z*-axis.

In a preferred variant, during each calibration measurement, averaging is applied; preferably, the number of averages is set for a targeted signal-to-noise-ratio SNR. Averaging means repetition of a measurement with identical parameters. Averaging is used to decrease the level of noise floor. In case of the trajectory specific system matrix as described above, the entire trajectory is repeated during a calibration measurement. In the case of the generic system matrix, short trajectory sections are repeated during a calibration measurement. In case SNR of the signal of a calibration measurement is not sufficient, the required number of averaging can be determined experimentally for a targeted SNR. SNR of a measured signal is ideally proportional to the square root of the number of averages applied, if the noise is random, uncorrelated, and stationary.

In a highly preferred variant, the calibration source is moved relative to the region of interest exclusively in the drive field direction, in order to move the calibration source between the target positions.

In a preferred variant, a fully sampled calibration data consists of $n$ measurements, the number of calibration measurements $m$, is smaller than the number $n$, and calibration data not measured is estimated, in particular by interpolation and/or extrapolation using the already acquired calibration data (undersampling). Due to geometrical symmetries, calibration data of some of the trajectory offsets can be generated using acquired calibration measurements, e.g. by phase shifting, amplitude scaling, and/or flipping.

For image reconstruction of MPI-data which have been acquired by performing an MPI sequence, a calculated multi-dimensional image-domain system matrix $\boldsymbol{A}$ is used, wherein a convex optimization algorithm is used which is based on a forward model, in particular the following:

$$\underset{\rho}{argmin} \ \|A\rho - b\|_2^2 + \lambda \, \Phi(\rho)$$

where

$A$ = calibration matrix, i.e. multi-dimensional image-domain system matrix,

$\rho$ = MPI-image

$b$ = measurement vector,

$\lambda$ = regularization constant for regularization operation

$\Phi(\cdot)$ = regularization operator.

When multiple receiver coils are used, the calibration matrices of each coil and measurement vectors of each coil can be merged (concatenated) prior to reconstruction. In other words:

$$\begin{bmatrix} A \text{ of receiver } 1 \\ A \text{ of receiver } 2 \\ \vdots \\ A \text{ of receiver } C \end{bmatrix} \rightarrow A \text{ and } \begin{bmatrix} b \text{ of receiver } 1 \\ b \text{ of receiver } 2 \\ \vdots \\ b \text{ of receiver } C \end{bmatrix} \rightarrow b$$

where $C$ is the number of receiver coils.

To improve the signal-to-noise ratio and/or image reconstruction accuracy, noisy frequency components can be removed from the multidimensional system matrices of the receiver coils and from the measurement vectors of the receiver coils. In this case, the multi-dimensional system matrices of the receiver coil and the measurement vectors of the receiver coils are reduced.

In a preferred variant, projections of MPI-image on the *xz*- or *yz*-plane at a specific angle $\theta_{\text{projection}}$ can be imaged by setting FFL angle $\theta_{traj}(t) = \theta_{\text{projection}}$ (a special variant of the sinusoidal trajectory). The result of image reconstruction, the MPI-image $\rho$, will be directly the projection of the MPI-image at the specific angle $\theta_{\text{projection}}$ without further post-processing.

In a preferred variant, (maximum intensity) projection of MPI image on any plane can be calculated by picking the maximal value or summing all slices of the reconstructed MPI-image $\rho$ along the selected plane after reconstruction.

Further advantages of the invention can be derived from the description and the drawings. Also, the above-mentioned and the still further described features can be used according to the invention individually or in any combination. The embodiments shown and described are not to be understood as a conclusive list, but rather have an exemplary character for the description of the invention.

Brief description of the drawings

| | |
|---|---|
| Fig. 1 | shows the basic method steps of the inventive method for generating a multi-dimensional image-domain system matrix using a multi-dimensional sinogram-based system matrix. |
| Fig. 2 | shows the steps of a **first variant** of generating the multi-dimensional sinogram-based system matrix using a trajectory specific calibration data acquisition by offsetting the trajectory. |
| Fig. 3a | shows the temporal course of FFL offset (r) of a 3D FFL sinusoidal trajectory. |
| Fig. 3b | shows the temporal course of FFL offset (r) of a 3D FFL sinusoidal trajectory with trajectory offset $\Delta r$. |
| Fig. 3c | shows the temporal course of FFL-angle, which does not depend on trajectory offset $\Delta r$, during the sinusoidal trajectory |
| Fig. 4 | shows the steps of a calibration data acquisition with varying FFL-offset for each source target position for the first variant. |
| Fig. 5 | shows the steps of a **second variant** of generating the multi-dimensional sinogram-based system matrix using a Magnetic Particle Spectrometer. |
| Fig. 6 | shows the steps of a **third variant** of generating the multi-dimensional sinogram-based system matrix using a generic system matrix. |
| Fig. 7 | shows a calibration volume with overscanning region with a superimposed trajectory. |
| Fig. 8a-c | show FFL-offset-positions for 1D source movement. |
| Fig. 9 | shows a region of interest and an FFL with its FFL-parameters. |
| Fig. 10a | shows the temporal course of a 3D FFL sinusoidal trajectory with 1D excitation. |
| Fig. 10b | shows the temporal course of the FFL-angle during the sinusoidal trajectory. |
| Fig. 10c | shows the sinusoidal trajectory of Fig. 10a in a sinogram. |
| Fig. 11a | shows a sinusoidal trajectory in sinogram-domain and image-domain. |
| Fig. 11b,c | shows offset sinusoidal trajectories in sinogram-domain and image-domain. |

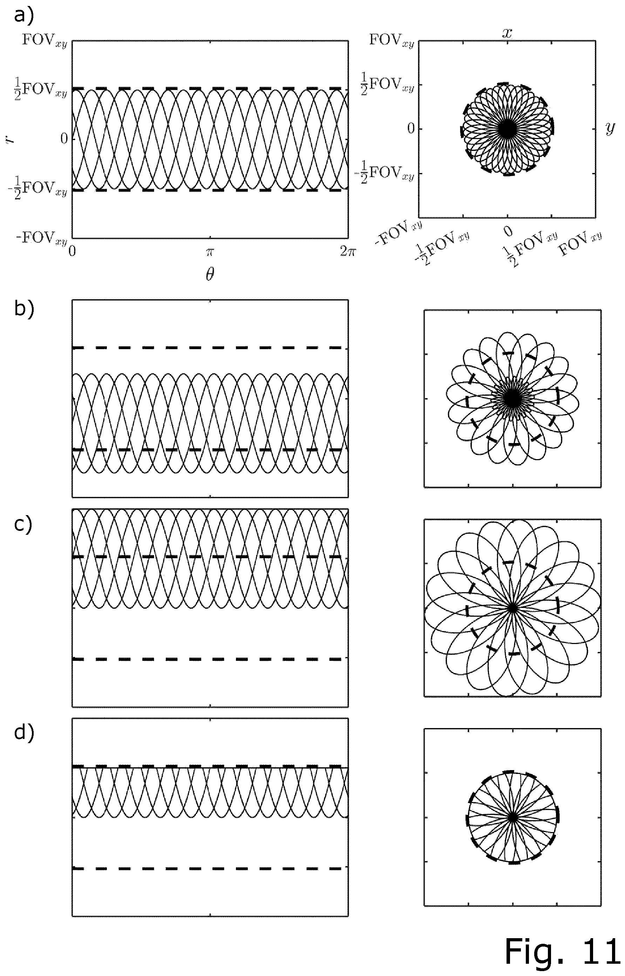

Fig. 11d      shows the offset sinusoidal trajectory of Fig. 11c clipped in sinogram-domain and image-domain.

Detailed description of the drawings

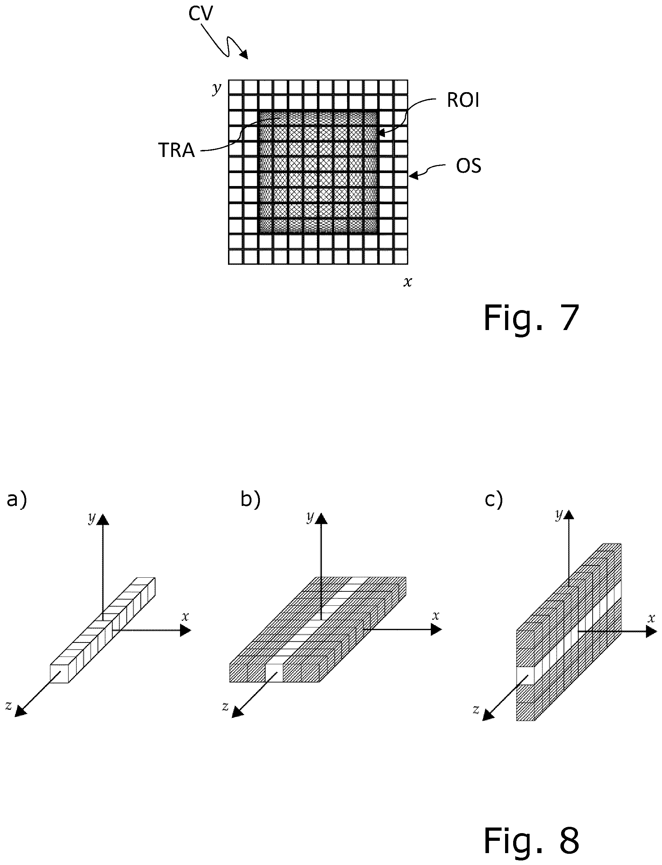

**Fig. 7** shows a calibration volume **CV** (image domain x, y) with a region of interest **ROI,** from which an MPI-image is to be generated, and an overscanning region **OS** (2D-illustration). In a 3D calibration volume CV, a third dimension z is added (not shown).

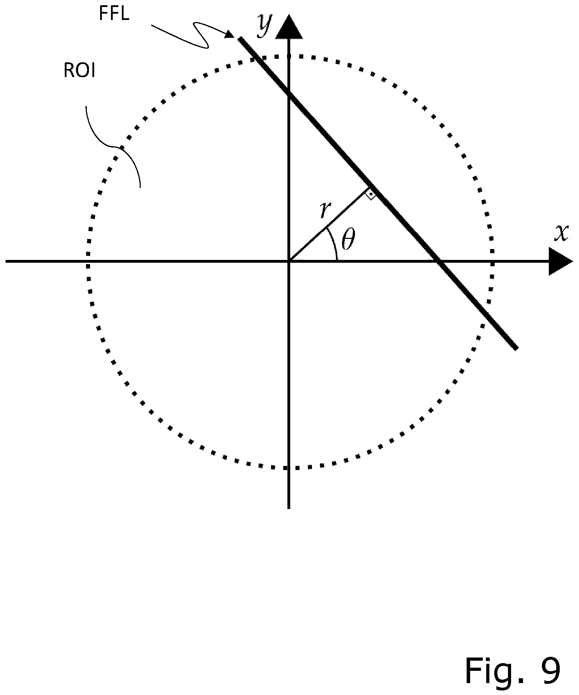

Within the region of interest, ROI a trajectory **TRA** is indicated as example along which a field free line **FFL** (see Fig. 9) is moved during applying an MPI-sequence. According to the prior art, the trajectory TRA is repeated for any calibration source position within the region of interest ROI. During tracing the trajectory TRA the FFL-angle, FFL-offset, FFL-angular-velocity, FFL-translational-velocity might change.

When using an FFL, the reconstruction cannot be performed directly to the image domain (x, y, z), but to a sinogram domain (θ, r, z):

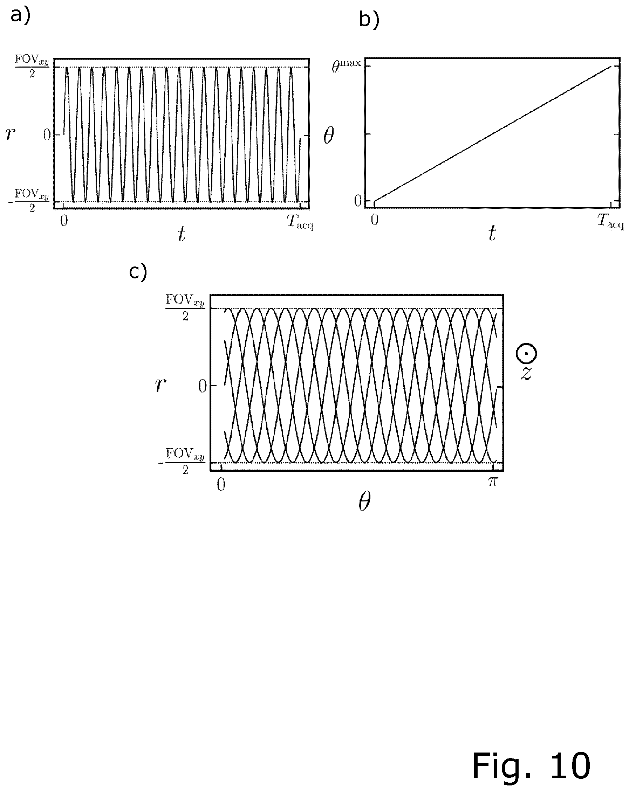

**Fig. 9** shows a field free line **FFL** in the region of interest ROI with an FFL-angle θ (angle between the FFL-orientation and the x-axis) and an FFL-offset **r** (distance of the FFL to the origin). During running a trajectory, the FFL is moved through the calibration volume and thus having different FFL-offsets during a trajectory run. Translation and rotation of the FFL take place within the x- and y-direction, which is defined by magnetic shift/focus fields and/or mechanical rotation. A 3D field of view FOV can be imaged by rotating and shifting the FFL in the x- and y-directions between drive field excitation cycles (step-wise) or during drive field excitation cycles (dynamically). In other words, by modulating the shift or focus fields to cover each (x,y) position, the field of view FOV can be traversed, for example, using a step-wise meander or dynamic sinusoidal trajectory, as shown in **Fig. 10** for the dynamic case. A translation (offset) of the FFL corresponds to moving vertically in the 2D sinogram, whereas a rotation of the FFL corresponds to moving horizontally. Moving in the z-direction (e.g., by DF excitation or focus/shift fields) corresponds to moving orthogonal to the 2D sinogram.

The fields in 3D generated with the exemplary dynamic sinusoidal trajectory with 1D excitation in the z-direction can be defined with the following equations:

$$\boldsymbol{H}(\theta) = G \begin{bmatrix} \frac{1}{2} + \frac{1}{2}\cos 2\theta & \frac{1}{2}\sin 2\theta & 0 \\ \frac{1}{2}\sin 2\theta & \frac{1}{2} - \frac{1}{2}\cos 2\theta & 0 \\ 0 & 0 & 1 \end{bmatrix} \begin{bmatrix} x \\ y \\ z \end{bmatrix}, \quad \theta = 2\pi f_{rot} t$$

$$\boldsymbol{H_{DF}}(t) = \begin{bmatrix} 0 \\ 0 \\ A_{DF} \sin(2\pi f_{DF} t) \end{bmatrix}$$

$$\boldsymbol{H_{FF}}(t) = \begin{bmatrix} H_{FF} \sin(2\pi f_{tra} t) \cos(2\pi f_{rot} t) \\ H_{FF} \sin(2\pi f_{tra} t) \sin(2\pi f_{rot} t) \\ 0 \end{bmatrix}, \quad H_{FF} = G \frac{FOV_{xy}}{2},$$

where $G$ is the gradient of the selection field, $A_{DF}$ and $f_{DF}$ are drive field amplitude and frequency, respectively, and $f_{rot}$ and $f_{tra}$ are the rotational and translational frequencies of the trajectory, respectively.

**Fig. 10 a-c** show the temporal course of a 3D FFL sinusoidal trajectory (Fig. 10a), the temporal course of the FFL-angle during the sinusoidal trajectory (Fig. 10b) and a sinogram representation of the sinusoidal trajectory (Fig. 10c), each with 1D DF-excitation orthogonal to the shown plane.

By using 1D excitation in a z-direction, a 3D FOV can be imaged by rotating and translating the FFL in the x- and γ-directions. This can be achieved, for example, with a sinusoidal trajectory as shown in Fig. 10, which can be described by the following equations:

$$r_{\text{traj}}(t) = \frac{FOV_{xy}}{2} \sin(2\pi f_{\text{tra}} t)$$

$$\theta_{\text{traj}}(t) = 2\pi f_{\text{rot}} t$$

where $FOV_{xy}$ is the FOV in the xy-plane, $t \in [0\ T_{\text{acq}}]$, $T_{\text{acq}}$ is the sequence duration, and $f_{\text{tra}}$ and $f_{\text{rot}}$ are the translational and rotational frequencies of the FFL, respectively. To ensure periodicity and the densest coverage in the sinogram representation of the trajectory shown in Fig. 10c for a given $T_{acq}$, $f_{tra}$ and $f_{rot}$ must be appropriately set.

According to the invention, a method is suggested which uses a multi-dimensional sinogram-based system matrix to generate a multidimensional image-domain system matrix, which is used for reconstruction of an MPI image from acquired MPI-data. The multi-dimensional sinogram-based system matrix is obtained by performing calibration measurements for $N_r$ offsets of the FFL or the FFL trajectory within the calibration volume. In contrast to conventional image-domain calibration, MPI sequence repetitions preferably comprise stepped target positions z, and FFL trajectory offsets (with identical FFL-angles) that are not along the FFL-directions, since it is assumed that the signals received from the spatial positions along the FFL are identical. Thus, the number of calibration measurements can be significantly reduced using this dimensionality reduction. As opposed to conventional image-domain calibrations which require repetitions for each voxel in 3D image-space (calling for a 3-axis robot), sinogram-based calibrations require repetitions for each FFL-offset in the offset dimension of a sinogram and target position $z$. For example, for a 3D image of size $[N_{xy}, N_{xy}, N_z]$, conventional image-domain calibration requires $N_{xy} \times N_{xy} \times N_z$ repetitions, whereas sinogram-based system matrix acquisition requires $N_{xy} \times N_z$ repetitions ($N_r = N_{xy}$), while allowing for field based trajectory offsets in e.g. xy-plane.

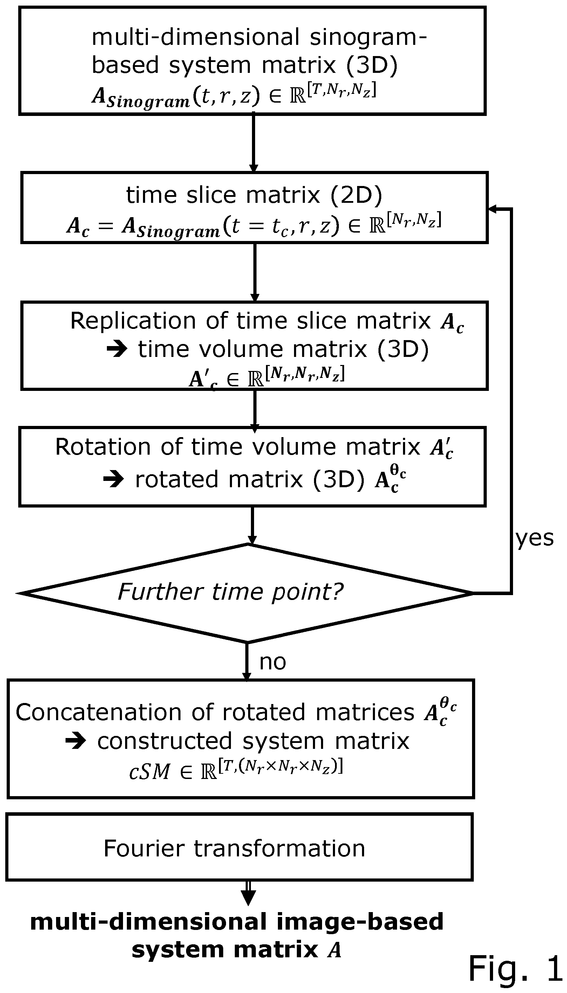

The basic method steps of this method are shown in **Fig. 1.** In the following, a 3D FOV is assumed (in the case of a 2D FOV, the z-dimension is omitted).

First, time slice matrices $A_c = A_{Sinogram}(t = t_c, r, z) \in \mathbb{R}^{[N_r, N_z]}$ are created from the multi-dimensional sinogram-based system matrix for specific time points tc of the trajectory, wherein each time point tc is specific for a time point within the trajectory. I.e. a specific time point tc occurs repeatedly, namely once each time the trajectory is run through. Each time slice matrix $A_c$ comprises data of the multi-dimensional sinogram-based system matrix $A_{Sinogram}$ for the specific time point (t= tc). Each time slice matrix $A_c$ is replicated $N_r$- 1 times, so that $N_r$ identical time slice matrices $A_c$ are obtained (one time slice matrix $A_c$ for each FFL-offset). The $N_r$ identical time slice matrices $A_c$ for the specific time point together form a time volume matrix $A'_c$. The time volume matrix $A'_c$ is then rotated by the FFL angle $\theta_c = \theta(t = t_c)$ of the FFL at the specific time point $t_c$, thereby resulting in a rotated matrix $A_c^{\theta_c}$ whose dimensions are associated with the spatial dimensions ($x, y, z$). A constructed system matrix cSM is obtained by concatenating all rotated matrices $A_c^{\theta_c}$ and by vectorising the spatial dimensions after concatenation using the vectorization operator $V$.

$$cSM = V \left\{ \begin{bmatrix} A_1^{\theta_1} \\ A_2^{\theta_2} \\ \vdots \\ A_T^{\theta_T} \end{bmatrix} \right\} = V \left\{ \begin{bmatrix} \mathcal{R}_{\theta_1}\{\Psi\{A_1\}\} \\ \mathcal{R}_{\theta_2}\{\Psi\{A_2\}\} \\ \vdots \\ \mathcal{R}_{\theta_T}\{\Psi\{A_T\}\} \end{bmatrix} \right\}$$

The multi-dimensional image-domain system matrix $A$ is obtained after performing a Fourier transformation on the constructed system matrix $cSM$ in its time-dimension.

$$A = \mathcal{F}\{cSM\}$$

In the following, three variants are described for obtaining the multi-dimensional sinogram-based system matrix $A_{Sinogram}$. With all described variants, it is possible to move the calibration source only in one direction within a 3D region of interest ROI (e.g. in z-direction, while staying at fixed position (preferably at the center of the ROI) within the xy-plane) or to not move the calibration source within a 2D region of interest ROI (e.g. at x=0, y=0 within the xy-plane). In order to nevertheless ensure that signals originating from off-centered positions (x≠0 | y≠0) are also taken into account in the generation of the system matrix, which is essential for accurate image reconstruction, calibration measurements with corresponding offsets are repeated in accordance with the invention. **Fig. 8a** shows positions of the calibration source used for calibration with 1D movement of the calibration source, here in the z-direction as example (e.g. for 1D excitation). **Fig. 8b** and **Fig. 8c** show offset-positions of the FFL (striped) for the case that an FFL with extension in y-direction for FFL-angle is 0° (Fig. 8b) and x-direction for FFL-angle is 90° (Fig. 8c) is used. The offsets are orthogonal to the extension of the FFL.

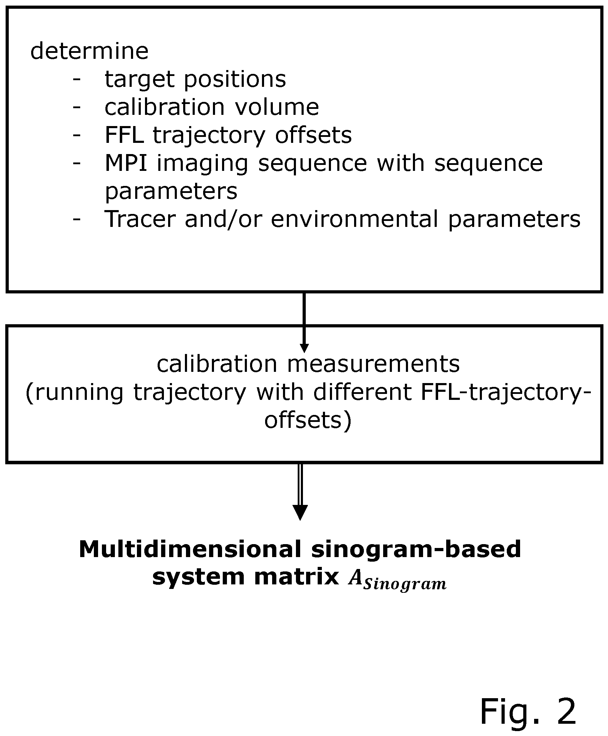

**Fig. 2** shows the method steps of a **first variant (trajectory offset variant)** of generating the multi-dimensional sinogram-based system matrix using a trajectory specific calibration data acquisition by offsetting the trajectory. Calibration measurements are performed in a sinogram domain that is specifically tailored to a given trajectory (e.g. the 3D FFL sinusoidal trajectory shown in Fig. 10a-c).

In contrast to the methods known from the prior art, the first variant of the inventive method does not comprise running a specific trajectory repeatedly for different target positions of the calibration source, but to shift the trajectory by FFL trajectory offsets, wherein for all FFL trajectory offsets, calibration data is acquired during running the trajectory.

First, relevant items required to perform the method are determined, in particular: MPI imaging sequence (trajectory), positions to which the calibration source is to be moved within the region of interest ROI (target positions), calibration volume within which the trajectory is to be offset (size, resolution), FFL trajectory offsets, tracer and environmental parameters, e.g. tracer, temperature of the tracer, viscosity of the tracer environment. The FFL trajectory is preferable not only shifted within the region of interest ROI but also to the overscanning region OS and acquisition window during which a calibration measurement is to be performed.

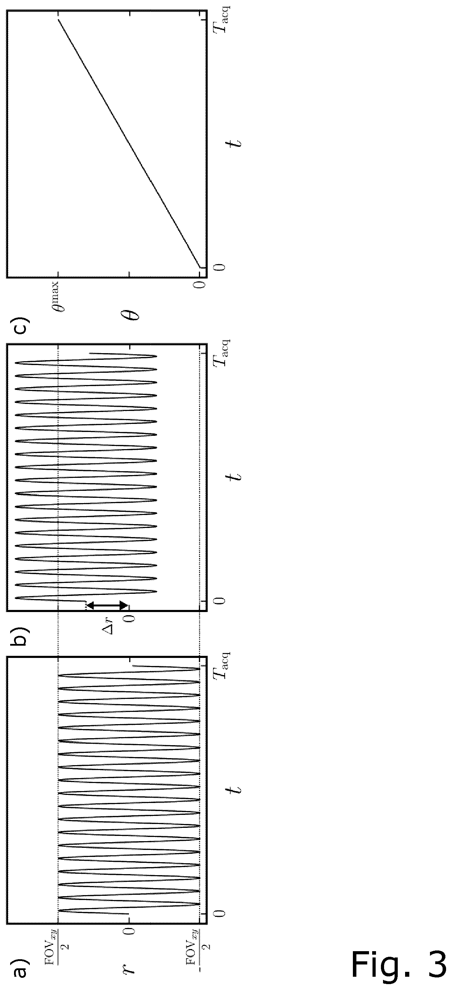

Calibration measurements are performed by running the trajectory, wherein during each calibration measurement the trajectory is run at least once with a specific FFL-trajectory-offset. The FFL-trajectory-offsets are constant during a calibration measurement. The FFL-trajectory-offsets are additional FFL-offsets to the trajectory. Thus, the additional FFL-offset is also always orthogonal to the extension of the FFL, also when the FFL angle changes during the trajectory; i.e. the direction of the additional FFL-offset due to the FFL-trajectory-offset rotates with the rotating FFL. The trajectories with FFL-trajectory-offsets are generated by using additional corresponding alternating (ac) magnetic focus fields. In order to perform the next calibration measurement, a new FFL trajectory offset is set, by using according alternating (ac) focus fields. **Fig. 3a,b** shows the sinusoidal trajectory shown in Fig. 10 for different FFL trajectory offsets ($\Delta$r=0 (Fig. 3a) and $\Delta$r= ¼FOV (Fig. 3b). The FFL-angle $\theta$ does not depend on FFL-trajectory-offset $\Delta$r. In other words, additional FFL-trajectory-offset $\Delta$r does not change the angle of the FFL for any given time (**Fig. 3c**).

**Fig. 11a-d** shows a sinusoidal trajectory in sinogram-domain and image-domain with trajectory offset $\Delta$r=0 (Fig. 11a), trajectory offset $\Delta$r=-¼FOV (Fig. 11b) and trajectory offset $\Delta$r=1/2 FOV (Fig. 11c). The dotted lines represent the boundaries of the FOV, which corresponds to the bore edge of the MPI scanner. Since no data can be acquired outside the FOV, the trajectory can be clipped as shown in Fig. 11d.

By using this sinogram-based calibration data for synthesizing the multidimensional image-domain system matrix in the image domain, the total calibration time is drastically lower than with conventional calibration:

The sinogram-based system calibration sequence for the 3D FFL sinusoidal trajectory shown in Fig. 10 is defined by the following equation:

$$r_k(t) = \frac{\text{FOV}_{xy}}{2}\sin(2\pi f_{\text{tra}}t) + \Delta r_k$$

where

$$\Delta r_k = -\frac{\text{FOV}_{xy}}{2} + k \cdot d_{xy}, \quad k \in \{0,1,...,N_{xy}-1\},$$

$$d_{xy} = \frac{\text{FOV}_{xy}}{N_{xy}},$$

and $N_{xy}$ are the voxel size and the number of voxels in the *xy*-directions, respectively. This procedure is repeated for each calibration source position of the tracer probe, preferably in the center of the MPI system bore (*x* = *y* = 0) along the *z*-direction:

$$z_l = -\frac{\text{FOV}_z}{2} + l \cdot d_z, \quad l \in \{0,1,...,N_z-1\},$$

$d_z = \frac{\text{FOV}_z}{N_z}$ and $N_z$ are the voxel size and the number of voxels in the z-direction, respectively. The total measurement time is therefore $N_{xy} \times N_z \times T_{\text{acq}}$, assuming 100% acquisition duty cycle. The resulting sinogram-based calibration data can be represented as $A_{sino}(t,r,z) \in \mathbb{R}^{T \times N_{xy} \times N_z}$, where *T* is the number of data points per measurement.

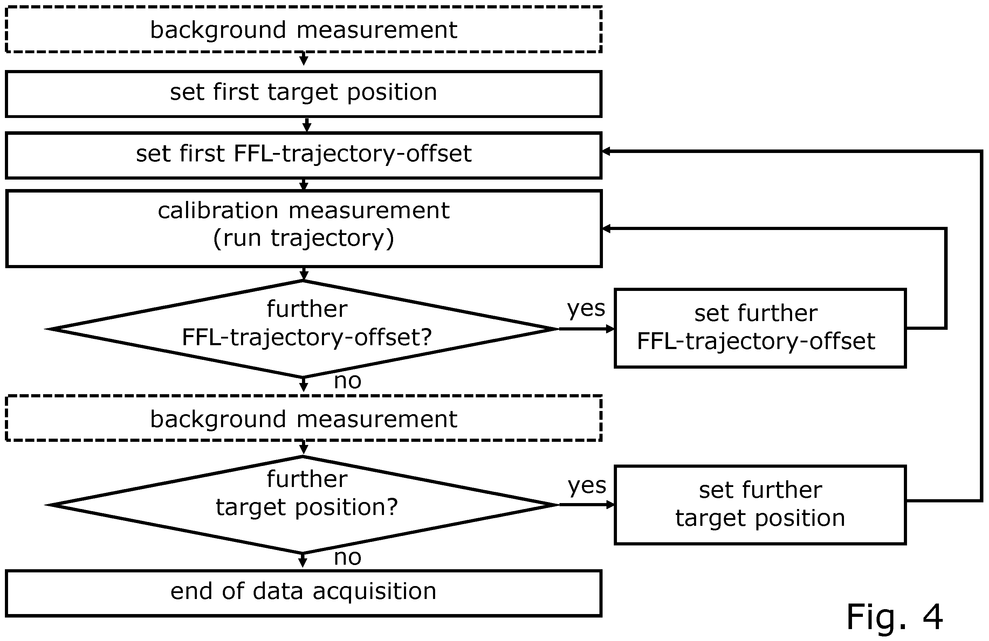

**Fig. 4** shows a preferred variant how to perform the calibration measurements. First, the calibration source is positioned in a first target position. Preferably, prior to positioning the calibration source, background measurements can be carried out. The field-free line is positioned at a first FFL-position by setting a first FFL trajectory offset. Starting from this FFL-position, one calibration measurement is performed by running the trajectory. This is repeated for each FFL trajectory offset. Then the calibration source is positioned at a further target position (e.g. by moving the calibration source mechanically) and the whole procedure is repeated for all target positions. Before setting each new target position, an optional background measurement is performed.

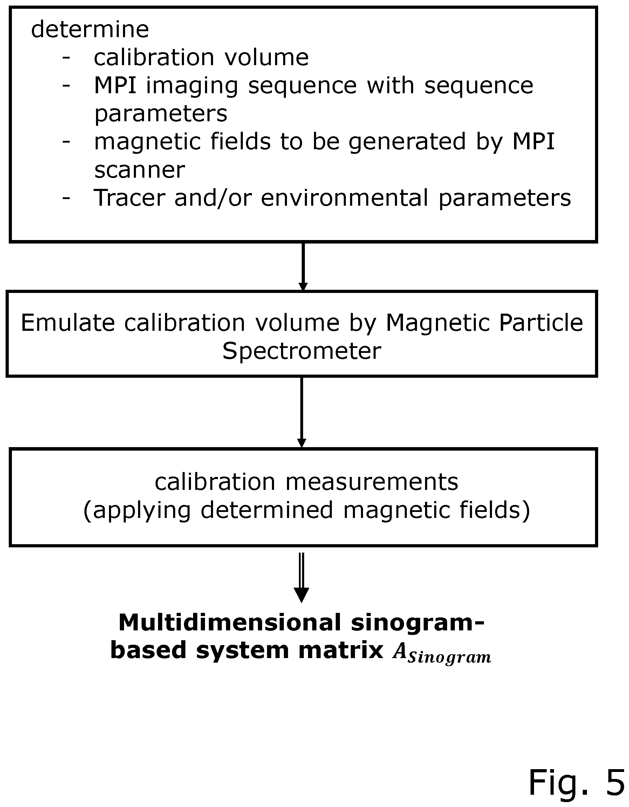

**Fig. 5** shows the method steps of a **second variant (MPS variant)** of generating the multi-dimensional sinogram-based system matrix using a Magnetic Particle Spectrometer for emulating the calibration volume.

In contrast to the methods known from the prior art, the second variant of the inventive method does not use 3D fields with gradients generated by an MPI scanner but a homogeneous field corresponding to a specific voxel position of MPI scanner at each time point generated by the Magnetic Particle Spectrometer. In this case, there is no magnetic field gradient and therefore no field free region.

First, relevant items required to perform the method are determined, in particular: Calibration volume of an MPI scanner in sinogram domain, from which calibration data is to be acquired, MPI scanner parameters (including drive field frequency, drive field amplitude, and magnetic field gradient G of an MPI scanner), MPI imaging sequence (trajectory), tracer and/or environmental parameters (e.g., temperature and viscosity), magnetic fields B(t, $\Delta r$, **z**) to be generated by an MPI scanner in order to emulate the magnetic fields of an FFL trajectory for only one voxel position (for each target position) of an MPI scanner. The calculated fields depend on the MPI scanner parameters and the trajectory. In other words: The magnetic fields that an MPI scanner would generate when running the determined MPI sequence are calculated. These calculated magnetic fields are then applied to the calibration source in the MPS.

The trajectory is preferably not only shifted within the region of interest ROI but also to the overscanning region OS and acquisition window during which a calibration measurement is to be performed.

Instead of moving the calibration source mechanically, the varying z-positions of the calibration source is emulated by applying additional constant (dc) fields in the z-direction using the Magnetic Particle Spectrometer.

By acquiring sinogram-based calibration data using MPS for synthesizing the multidimensional system matrix in the image domain, the MPI scanner is not blocked during calibration measurements, reducing the downtime of the imaging device. Moreover, signals acquired with MPS can have significantly higher SNR, allowing generation of system matrices with higher spatial resolution and more accuracy.

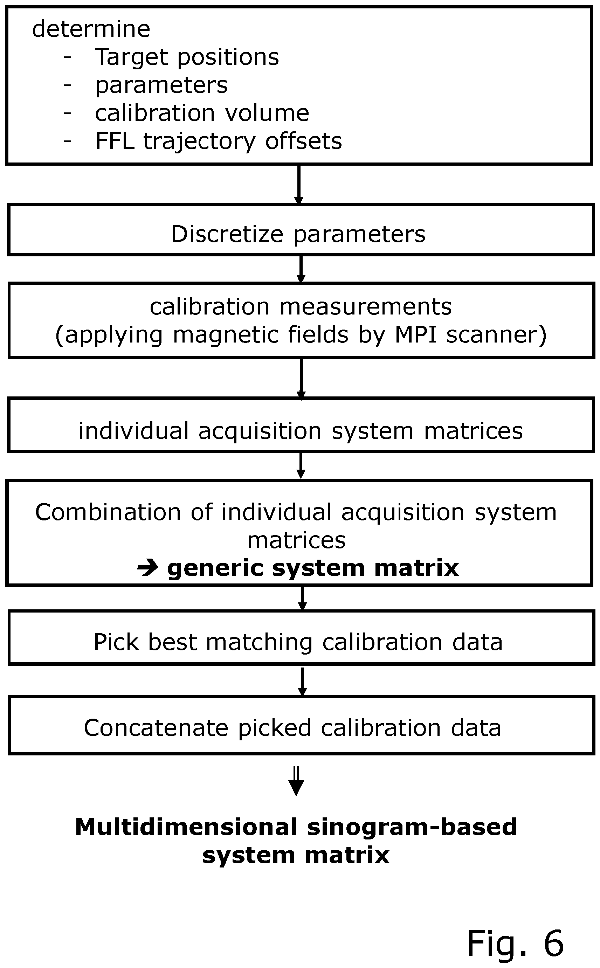

**Fig. 6** shows the method steps of a **third variant (generic variant)** of generating the multi-dimensional sinogram-based system matrix using a generic calibration data acquisition method independent of a specific trajectory.

In contrast to the methods known from the prior art, the third variant of the inventive method does not comprise running a specific trajectory repeatedly for different target positions of the calibration source, but to shift the FFL and acquire data with various parameter value combinations for the respective FFL position, wherein calibration data is acquired for all FFL-offsets and all set parameter value combinations.

First, relevant items required to perform the method are determined, in particular: positions to which the calibration source is to be moved within the region of interest ROI (target positions), calibration voxels from which signals are to be acquired (calibration voxels), parameters to be varied in order to get information for different parameter value combinations, positions to which the FFL is to be shifted by setting respective FFL-offsets. The FFL is preferable not only shifted within the region of interest ROI but also to the overscanning region OS and acquisition window during which a calibration measurement is to be performed.

In contrast to running complete trajectories (as e.g. in variant 1), the parameters are discretized before the calibration measurements are performed. After the calibration measurements, individual acquisition system matrices (with dimensions frequency (or time) and FFR-offset) are determined. By combining the individual system matrices $A_c$ the multidimensional system matrix **A** is acquired. For example, for the multidimensional system matrix **A** with dimensions frequency, FFL-offset, calibration source positions z along the z-direction at the center of the ROI ($x = y = 0$), FFL velocity, and FFL angle, can be represented in terms of is individual acquisition system matrices $A_c$ as following:

$$\boldsymbol{A}(f, r, z, v = v_c, \theta = \theta_c) = A_c(f, r, z)$$

where the individual system matrices $A_c$ is acquired for FFL velocity $v_c$ and FFL angle $\theta_c$.

From the generic system matrix the best matching calibration data (i.e. calibration data of that calibration measurement that best matches to the combination of values of the predetermined parameters of the respective drive field cycle of an MPI measurement from which an MPI image is to be reconstructed) is picked and concatenated, thereby obtaining the multi-dimensional sinogram-based system matrix.

In contrast to the methods known from the prior art, the third variant of the inventive method does not comprise running a specific trajectory repeatedly for different target positions of the calibration source, but provides for shifting the FFL to predefined calibration voxels and setting a multitude of parameter value combinations, wherein a calibration data is acquired for all FFL-offsets and all set parameter value combinations.

List of reference signs

CV     calibration volume

ROI    region of interest

OS     overscanning region

T     trajectory

FFL    field free line

<u>List of cited documents</u>

| | |
|---|---|
| [Weizenecker2008] | Weizenecker et al. "Magnetic particle imaging using a field free line" Journal of Physics D: Applied Physics 41(10): 105009, 2008, doi: 10.1088/0022-3727/41/10/105009. |
| [Weizenecker2009] | Weizenecker et al. "Three-dimensional real-time in vivo magnetic particle imaging" Physics in Medicine & Biology 54(5):L1, 2009, doi: 10.1088/0031-9155/54/5/L01. |
| [Kaethner] | Kaethner et al., "Axially Elongated Field-Free Point Data Acquisition in Magnetic Particle Imaging," IEEE Transactions on Medical Imaging, vol. 34, no. 2, pp. 381-387, Feb. 2015, doi: 10.1109/TMI.2014.2357077. |
| [Von Gladiss] | Von Gladiss, A., et al. "Hybrid system calibration for multidimensional magnetic particle imaging." Physics in Medicine & Biology 62.9 (2017): 3392 |
| [Halkola] | Halkola et al. "System Calibration Unit for Magnetic Particle Imaging: Focus Field Based System Function" Buzug, T., Borgert, J. (eds) Magnetic Particle Imaging. Springer Proceedings in Physics, vol 140. Springer, Berlin, Heidelberg. doi: 10.1007/978-3-642-24133-8_5 |
| [Li] | Li et al. "Fast System Matrix Generation Based on Single Angle Calibration in Open-Sided Field Free Line Magnetic Particle Imaging" IEEE TRANSACTIONS ON BIOMEDICAL ENGINEERING, VOL. 71, NO. 4, APRIL 2024, p. 1209-1218 |
| [Mohn] | Mohn et al. "System Matrix Based Reconstruction for Pulsed Sequences in Magnetic Particle Imaging" IEEE TRANSACTIONS ON MEDICAL IMAGING, VOL. 41, NO. 7, JULY 2022, p. 1862-1873 |
| [CN115797493] | CN115797493 |
| [US2015221103] | US 2015 221 103. |
| [US20170020407] | US20170020407A1 |
| [US009651637] | US009651637B2 |

**Claims**

1.  Method for generating a multi-dimensional image-domain system matrix *A* for MPI measurements performed by an MPI Scanner using an MPI-sequence with a field free line (FFL) with FFL angles $\theta$, FFL offsets *r*, and FFL velocities,

the method using a multi-dimensional sinogram-based system matrix $A_{Sinogram}(t,r,z) \in \mathbb{R}^{[T,N_r,N_z]}$, acquired from calibration measurements, the sinogram having an offset-dimension, an angle-dimension, and a *z*-dimension,

*r* = FFL-offset in the offset-dimension of the sinogram
$\theta$ = angle in the angle-dimension of the sinogram
*t* = time in the time-dimension of the multi-dimensional sinogram-based system matrix $A_{Sinogram}$ and multi-dimensional image-domain system matrix *A*
$N_r$ = number of FFL-offsets
$N_z$ = number of voxels of a region of interest (ROI) in a *z*-direction
*T* = number of time points of the time dimension (number of calibration data points during an MPI imaging sequence)

wherein the method comprises the following steps:

  • for each time point $t_c$, $c \in [1,T]$:

◦ get a time slice matrix $A_c = A_{Sinogram}(t = t_c, r, z) \in \mathbb{R}^{[N_r, N_z]}$ , which comprises calibration data of the multi-dimensional sinogram-based system matrix $A_{sinogram}(t, r, z)$ at time point $t = t_c$, the time slice matrix having a 1st offset-dimension r and a z-dimension *z*,

◦ replicate the time slice matrix $A_c$ $N_r$ - 1 times, resulting in $N_r$ identical time slice matrices $A_c$, and copying these replications along a newly added 2nd offset-dimension, resulting in a time volume matrix $A'_c \in \mathbb{R}^{[N_r, N_r, N_z]}$ ,

◦ rotate the time volume matrix $A'_c$ by the FFL angle $\theta_c = \theta(t = t_c)$ of the respective field free line (FFL) at time point $t = t_c$ in its 1st and 2nd offset-dimensions, resulting in a rotated matrix $A_c^{\theta_c}$ , where the dimensions of $A_c^{\theta_c}$ are associated with the spatial dimensions (*x*, *y*, *z*),

• concatenate the rotated matrices $A_c^{\theta_c}$ of all time points along a time-dimension and vectorize the spatial dimensions (*x*, *y*, *z*), resulting in a constructed system matrix $cSM$ $cSM \in \mathbb{R}^{[T, (N_r \times N_r \times N_z)]}$ ,

• perform a Fourier transformation on the constructed system matrix $cSM$ on its time-dimension resulting in the multi-dimensional image-domain system matrix *A*.

2. Method according to claim 1, **characterized in that** the multi-dimensional sinogram-based system matrix $A_{Sinogram}(t, r, z) \in \mathbb{R}^{[T, N_r, N_z]}$ is generated according to the following method:

• prior to calibration measurements, determine:

- target positions for a calibration source,
- a calibration volume (CV) in sinogram domain from which calibration data is to be acquired, the calibration volume (CV) comprising the region of interest (ROI),
- MPI imaging sequence with a trajectory (TRA) comprising sequence parameters, the sequence parameters including FFL angular frequency, FFL translational frequency, and MPI imaging sequence duration,
- Tracer and/or environmental parameters,
- FFL-trajectory-offsets Δ*r*, by which the positions of the field free line (FFL) during the trajectory (TRA) are to be shifted within the calibration volume (CV) perpendicular to the respective orientation of the field free line (FFL),

• perform calibration measurements by running the MPI imaging sequence at least once during each calibration measurement with a specific FFL-trajectory offset and a specific target position *z* using the MPI scanner, wherein the calibration measurements differ in the combination of FFL-trajectory-offset Δ*r* and target position *z*,
• determine the multi-dimensional sinogram-based system matrix $A_{Sinogram}$ from the calibration data.

3. Method according to claim 2, **characterized in that** performing the calibration measurements comprises the following steps:

i. the calibration source is positioned within the calibration volume (CV) at a first target position,
ii. calibration data is acquired with the calibration source being positioned at a first target position are performed, wherein the trajectory (TRA) of the MPI sequence is run through until all FFL-trajectory-offsets Δr have been set, wherein the FFL-trajectory-offset Δr is changed after the trajectory (TRA) is run through,
iii. the calibration source is positioned within the region of interest (ROI) at a further target position that is shifted relative to the previous target position within the calibration volume (CV) in at least one direction, preferably along a drive field direction, and
iv. the trajectory (TRA) of the MPI sequence is run through until all FFL-trajectory-offsets Δr have been set, wherein the FFL-trajectory-offset Δr is changed after the trajectory (TRA) is run through with the calibration source being positioned at the further target position,
v. steps iii) - iv) are repeated until measurements for all target positions of the calibration source are performed.

4. Method according to claim 3, **characterized in that** background measurements are performed, preferably prior to positioning the calibration source at the target positions in step i and step iii, without the calibration source being in the calibration volume (CV) and/or with the calibration source being in a magnetically saturated state.

5. Method according to claim 1, **characterized in that** the multi-dimensional sinogram-based system matrix $A_{Sinogram}(t, r, z) \in \mathbb{R}^{[T, N_r, N_z]}$ is generated according to the following method using a Magnetic Particle Spectrometer for emulating a calibration volume (CV) the method comprising:

- prior to calibration measurements, determine:

  - calibration volume (CV) in sinogram domain, the calibration volume (CV) from which calibration data is to be acquired, the calibration volume (CV) comprising the region of interest (ROI),
  - MPI scanner parameters of the MPI scanner including drive field frequency, drive field amplitude, and magnetic field gradient G,
  - MPI imaging sequence with a trajectory (TRA) comprising sequence parameters, the sequence parameters including FFL angular frequency, FFL translational frequency, and MPI imaging sequence duration,
  - Tracer and/or environmental parameters,
  - FFL-trajectory-offsets $\Delta r$,
  - spatial positions z of the calibration volume (CV),
  - for each combination of FFL-trajectory-offset $\Delta r$ and spatial position z: time dependent magnetic fields B(t, $\Delta r$, $z$), wherein each magnetic field B(t, $\Delta r$, $z$) corresponds to the magnetic field generated by the MPI scanner during a trajectory run with a specific FFL trajectory offset $\Delta r$ at a specific spatial position z,

- perform calibration measurements by emulating the calibration volume (CV) by applying the determined magnetic fields B(t, $\Delta r$, $z$) to a calibration source,
  wherein for each combination of FFL-trajectory-offset $\Delta r$ and spatial position $z$ a calibration measurement is performed during which the corresponding magnetic field B (t, $\Delta r$, $z$) is applied at least once, and calibration data are acquired,
- determine the multi-dimensional sinogram-based system matrix $A_{Sinogram}$ from the calibration data.

6. Method according to claim 1, **characterized in that** the multi-dimensional sinogram-based system matrix $A_{Sinogram}(t, r, z) \in \mathbb{R}^{[T, N_r, N_z]}$ is generated according to the following method, wherein the offset is an FFL-offset:

- perform generic MPI calibration measurements by applying at least one drive field cycle of a drive field in each calibration measurement using an MPI scanner, wherein during each generic MPI calibration measurement data is acquired for a specific combination of values of target position for a calibration source, FFL offset, and of predetermined parameters, comprising FFL angle and FFL velocities,
- determine a generic system matrix $A_{generic}$, where $A_{generic}$ comprises calibration data acquired from the generic MPI calibration measurements, and

that trajectory-specific calibration data is selected from the generic system matrix $A_{generic}$ according to the following method:

- for each drive field cycle of an MPI measurement

  ○ determine the FFL angle, FFL offset, and FFL velocity,
  ○ pick the calibration data from the generic system matrix $A_{generic}$ of that calibration measurement that best matches to the combination of values of the predetermined parameters of the respective drive field cycle, and

- concatenate all picked calibration data along the time dimension, resulting in the multi-dimensional sinogram-based system matrix $A_{Sinogram}$.

7. Method according to claim 6, **characterized in that** the determined parameters comprise at least one of: particle type of used tracer of the calibration source, temperature of the calibration source, viscosity of the calibration source, velocity of the calibration source, drive field frequency, drive field amplitude, magnetic field gradient of an MPI system used for the MPI measurements, duration of the trajectory (TRA), position of optional hardware inserts, shape of the field free line (FFL), receiver coils used, and receiver coil orientations.

8. Method according to any of the preceding claims, **characterized in that** for setting a specific FFL-trajectory-offset $\Delta r$ of the MPI sequence or for setting a specific FFL offset $\Delta r$ the field free line (FFL) is shifted in the offset-dimension of the sinogram by applying additional focus fields.

9. Method according to any of the preceding claims, **characterized in that** multi-dimensional sinogram-based system matrix $A_{Sinogram}$ is clipped in at least one spatial dimensions ($r$, $z$) of the multi-dimensional sinogram-based system matrix $A_{sinogram}$, such that the multi-dimensional sinogram-based system matrix $A_{sinogram}$, only comprises calibration data from field offsets lower than a threshold $r_{lim}, z_{lim}$.

10. Method according to any of the preceding claims, **characterized in that** during each calibration measurement, averaging is applied, preferably the number of averages is set for a targeted signal-to-noise-ratio SNR.

11. Method according to any of the preceding claims **characterized in that** in order to move the calibration source between the target positions, the calibration source is moved relative to the region of interest (ROI) exclusively in the drive field direction.

12. Method according to any of the preceding claims, **characterized in that** a fully-sampled calibration data consists of $n$ measurements, that the number of calibration measurements $m$, is smaller than the number $n$, and that calibration data not measured is estimated, in particular by interpolation and/or extrapolation using the already acquired calibration data.

13. Method for image reconstruction using a calculated multi-dimensional image-domain system matrix $A$ according to anyone of claims 1 to 12, by providing an MPI measurement b and calculating the concentration of the particles with A and b.

14. Method for image reconstruction according to claim 13, **characterized in that** an iterative reconstruction algorithm is used for calculating the concentration of the particles, based on a forward model, in particular the following:

$$\underset{\rho}{\arg\min} \ \|A\rho - b\|_2^2 + \lambda \ \Phi(\rho)$$

where

$A$ = multi-dimensional image-domain system matrix,
$\rho$ = MPI-image
$b$ = measurement vector,
$\lambda$ = regularization constant for regularization operation, and
$\Phi(\cdot)$ = regularization operator.

**Amended claims in accordance with Rule 137(2) EPC.**

1. **Method** for generating a multi-dimensional image-domain system matrix A for MPI measurements performed by an MPI Scanner using an MPI-sequence with a field free line (FFL) with FFL angles $\theta$, FFL offsets r, and FFL velocities,

the method using a multi-dimensional sinogram-based system matrix $A_{Sinogram}(t, r, z) \in \mathbb{R}^{[T, N_r, N_z]}$ , acquired from calibration measurements, the sinogram having an offset-dimension, an angle-dimension, and a z-dimension,

$r$ = FFL-offset in the offset-dimension of the sinogram
$\theta$ = angle in the angle-dimension of the sinogram
$t$ = time in the time-dimension of the multi-dimensional sinogram-based system matrix $A_{Sinogram}$ and multi-dimensional image-domain system matrix $A$
$N_r$ = number of FFL-offsets
$N_z$ = number of voxels of a region of interest (ROI) in a z-direction
$T$ = number of time points of the time dimension (number of calibration data points during an MPI imaging sequence)

wherein the method comprises the following steps:

• for each time point $t_c$, $c \in [1, T]$:

○ get a time slice matrix $A_c = A_{Sinogram}(t = t_c, r, z) \in \mathbb{R}^{[N_r, N_z]}$, which comprises calibration data of the multi-dimensional sinogram-based system matrix $A_{Sinogram}(t,r,z)$ at time point $t = t_c$, the time slice matrix having a 1st offset-dimension $r$ and a z-dimension $z$,

○ replicate the time slice matrix $A_c$ $N_r$ - 1 times, resulting in $N_r$ identical time slice matrices $A_c$, and copying these replications along a newly added 2nd offset-dimension, resulting in a time volume matrix $A'_c \in \mathbb{R}^{[N_r, N_r, N_z]}$,

○ rotate the time volume matrix $A'_c$ by the FFL angle $\theta_c = \theta(t = t_c)$ of the respective field free line (FFL) at time point $t = t_c$ in its 1st and 2nd offset-dimensions, resulting in a rotated matrix $A_c^{\theta_c}$, where the dimensions of $A_c^{\theta_c}$ are associated with the spatial dimensions ($x$,$y$,$z$),

• concatenate the rotated matrices $A_c^{\theta_c}$ of all time points along a time-dimension and vectorize the spatial dimensions (x,y,z), resulting in a constructed system matrix $cSM \in \mathbb{R}^{[T, (N_r \times N_r \times N_z)]}$,

• perform a Fourier transformation on the constructed system matrix $cSM$ on its time-dimension resulting in the multi-dimensional image-domain system matrix A.

wherein the multi-dimensional sinogram-based system matrix $A_{Sinogram}(t, r, z) \in \mathbb{R}^{[T, N_r, N_z]}$ is generated according to the following method:

• prior to calibration measurements, determine:

- target positions for a calibration source,
- a calibration volume (CV) in sinogram domain from which calibration data is to be acquired, the calibration volume (CV) comprising the region of interest (ROI),
- MPI imaging sequence with a trajectory (TRA) comprising sequence parameters, the sequence parameters including FFL angular frequency, FFL translational frequency, and MPI imaging sequence duration,
- Tracer and/or environmental parameters,
- FFL-trajectory-offsets $\Delta r$, by which the positions of the field free line (FFL) during the trajectory (TRA) are to be shifted within the calibration volume (CV) perpendicular to the respective orientation of the field free line (FFL),

• perform calibration measurements by running the MPI imaging sequence at least once during each calibration measurement with a specific FFL-trajectory offset and a specific target position z using the MPI scanner, wherein the calibration measurements differ in the combination of FFL-trajectory-offset $\Delta r$ and target position z, wherein FFL-rotation and FFL-translation occur in the xy-plane,
• determine the multi-dimensional sinogram-based system matrix $A_{Sinogram}$ from the calibration data.

2. Method according to claim 1, **characterized in that** performing the calibration measurements comprises the following steps:

i. the calibration source is positioned within the calibration volume (CV) at a first target position,
ii. calibration data is acquired with the calibration source being positioned at a first target position are performed, wherein the trajectory (TRA) of the MPI sequence is run through until all FFL-trajectory-offsets $\Delta r$ have been set, wherein the FFL-trajectory-offset $\Delta r$ is changed after the trajectory (TRA) is run through,
iii. the calibration source is positioned within the region of interest (ROI) at a further target position that is shifted relative to the previous target position within the calibration volume (CV) in at least one direction, preferably along a drive field direction, and
iv. the trajectory (TRA) of the MPI sequence is run through until all FFL-trajectory-offsets $\Delta r$ have been set, wherein the FFL-trajectory-offset $\Delta r$ is changed after the trajectory (TRA) is run through with the calibration source being positioned at the further target position,

v. steps iii) - iv) are repeated until measurements for all target positions of the calibration source are performed.

3. Method according to claim 2, **characterized in that** background measurements are performed, preferably prior to positioning the calibration source at the target positions in step i and step iii, without the calibration source being in the calibration volume (CV) and/or with the calibration source being in a magnetically saturated state.

4. Method for generating a multi-dimensional image-domain system matrix $A$ for MPI measurements performed by an MPI Scanner using an MPI-sequence with a field free line (FFL) with FFL angles $\theta$, FFL offsets $r$, and FFL velocities,

the method using a multi-dimensional sinogram-based system matrix $A_{Sinogram}(t, r, z) \in \mathbb{R}^{[T, N_r, N_z]}$ , acquired from calibration measurements, the sinogram having an offset-dimension, an angle-dimension, and a $z$-dimension,

$r$ = FFL-offset in the offset-dimension of the sinogram
$\theta$ = angle in the angle-dimension of the sinogram
$t$ = time in the time-dimension of the multi-dimensional sinogram-based system matrix $A_{Sinogram}$ and multi-dimensional image-domain system matrix $A$
$N_r$ = number of FFL-offsets
$N_z$ = number of voxels of a region of interest (ROI) in a $z$-direction
$T$ = number of time points of the time dimension (number of calibration data points during an MPI imaging sequence)

wherein the method comprises the following steps:

• for each time point $t_c$, $c \in [1, T]$:

 ∘ get a time slice matrix $A_c = A_{Sinogram}(t = t_c, r, z) \in \mathbb{R}^{[N_r, N_z]}$ , which comprises calibration data of the multi-dimensional sinogram-based system matrix $A_{Sinogram}(t, r, z)$ at time point $t = t_c$, the time slice matrix having a 1st offset-dimension r and a z-dimension z,
 ∘ replicate the time slice matrix $A_c$ $N_r$ - 1 times, resulting in $N_r$ identical time slice matrices $A_c$, and copying these replications along a newly added 2nd offset-dimension, resulting in a time volume matrix $A_c' \in \mathbb{R}^{[N_r, N_r, N_z]}$ ,

 ∘ rotate the time volume matrix $A_c'$ by the FFL angle $\theta_c = \theta(t = t_c)$ of the respective field free line (FFL) at time point $t = t_c$ in its 1st and 2nd offset-dimensions, resulting in a rotated matrix $A_c^{\theta_c}$ , where the dimensions of $A_c^{\theta_c}$ are associated with the spatial dimensions $(x, y, z)$,

• concatenate the rotated matrices $A_c^{\theta_c}$ of all time points along a time-dimension and vectorize the spatial dimensions $(x, y, z)$, resulting in a constructed system matrix $cSM \in \mathbb{R}^{[T, (N_r \times N_r \times N_z)]}$ ,
• perform a Fourier transformation on the constructed system matrix $cSM$ on its time-dimension resulting in the multi-dimensional image-domain system matrix $A$.

wherein the multi-dimensional sinogram-based system matrix $A_{Sinogram}(t, r, z) \in \mathbb{R}^{[T, N_r, N_z]}$ is generated according to the following method using a Magnetic Particle Spectrometer for emulating a calibration volume (CV) the method comprising:

• prior to calibration measurements, determine:

 - calibration volume (CV) in sinogram domain, the calibration volume (CV) from which calibration data is to be acquired, the calibration volume (CV) comprising the region of interest (ROI),
- MPI scanner parameters of the MPI scanner including drive field frequency, drive field amplitude, and magnetic field gradient G,
- MPI imaging sequence with a trajectory (TRA) comprising sequence parameters, the sequence parameters including FFL angular frequency, FFL translational frequency, and MPI imaging sequence duration,
- Tracer and/or environmental parameters,
- FFL-trajectory-offsets $\Delta r$,
- spatial positions z of the calibration volume (CV),
- for each combination of FFL-trajectory-offset $\Delta r$ and spatial position z: time dependent magnetic fields B(t, $\Delta r$, z), wherein each magnetic field B(t, $\Delta r$, z) corresponds to the magnetic field generated by the MPI scanner during a trajectory run with a specific FFL trajectory offset $\Delta r$ at a specific spatial position z,

• perform calibration measurements by emulating the calibration volume (CV) by applying the determined magnetic fields B(t, $\Delta r$, z) to a calibration source,
wherein for each combination of FFL-trajectory-offset $\Delta r$ and spatial position *z* a calibration measurement is performed during which the corresponding magnetic field B (t, $\Delta r$, z) is applied at least once, and calibration data are acquired, wherein FFL-rotation and FFL-translation occur in the *xy*-plane,
• determine the multi-dimensional sinogram-based system matrix $A_{Sinogram}$ from the calibration data.

5. Method according to any of the preceding claims, **characterized in that** for setting a specific FFL-trajectory-offset $\Delta r$ of the MPI sequence or for setting a specific FFL offset $\Delta r$ the field free line (FFL) is shifted in the offset-dimension of the sinogram by applying additional focus fields.

6. Method according to any of the preceding claims, **characterized in that** multi-dimensional sinogram-based system matrix $A_{Sinogram}$ is clipped in at least one spatial dimensions ($r,z$) of the multi-dimensional sinogram-based system matrix $A_{Sinogram}$, such that the multi-dimensional sinogram-based system matrix $A_{Sinogram}$, only comprises calibration data from field offsets lower than a threshold $r_{lim}$, $z_{lim}$.

7. Method according to any of the preceding claims, **characterized in that** during each calibration measurement, averaging is applied, preferably the number of averages is set for a targeted signal-to-noise-ratio SNR.

8. Method according to any of the preceding claims **characterized in that** in order to move the calibration source between the target positions, the calibration source is moved relative to the region of interest (ROI) exclusively in the drive field direction.

9. Method according to any of the preceding claims, **characterized in that** a fully-sampled calibration data consists of *n* measurements, that the number of calibration measurements *m*, is smaller than the number *n,* and that calibration data not measured is estimated, in particular by interpolation and/or extrapolation using the already acquired calibration data.

10. Method for image reconstruction using a calculated multi-dimensional image-domain system matrix A according to anyone of claims 1 to 12, by providing an MPI measurement b and calculating the concentration of the particles with A and b.

11. Method for image reconstruction according to claim 10, **characterized in that** an iterative reconstruction algorithm is used for calculating the concentration of the particles, based on a forward model, in particular the following:

$$\underset{\rho}{\arg\min} \ \|A\rho - b\|_2^2 + \lambda\,\Phi(\rho)$$

where

$A$ = multi-dimensional image-domain system matrix,
$\rho$ = MPI-image
$b$ = measurement vector,
$\lambda$ = regularization constant for regularization operation, and

$\Phi(\cdot)$ = regularization operator.

multi-dimensional sinogram-
based system matrix (3D)
$$A_{Sinogram}(t, r, z) \in \mathbb{R}^{[T, N_r, N_z]}$$

time slice matrix (2D)
$$A_c = A_{Sinogram}(t = t_c, r, z) \in \mathbb{R}^{[N_r, N_z]}$$

Replication of time slice matrix $A_c$
➔ time volume matrix (3D)
$$A'_c \in \mathbb{R}^{[N_r, N_r, N_z]}$$

Rotation of time volume matrix $A'_c$
➔ rotated matrix (3D) $A_c^{\theta_c}$

Further time point?

yes

no

Concatenation of rotated matrices $A_c^{\theta_c}$
➔ constructed system matrix
$$cSM \in \mathbb{R}^{[T, (N_r \times N_r \times N_z)]}$$

Fourier transformation

**multi-dimensional image-based
system matrix $A$**

Fig. 1

determine
- target positions
- calibration volume
- FFL trajectory offsets
- MPI imaging sequence with sequence parameters
- Tracer and/or environmental parameters

calibration measurements
(running trajectory with different FFL-trajectory-offsets)

**Multidimensional sinogram-based system matrix** $A_{Sinogram}$

Fig. 2

Fig. 3

```
┌ ─ ─ ─ ─ ─ ─ ─ ─ ─ ─ ─ ─ ─ ─ ─ ─ ─ ─ ─ ─ ─ ─ ┐
│          background measurement             │
└ ─ ─ ─ ─ ─ ─ ─ ─ ─ ─ ─ ─ ─ ─ ─ ─ ─ ─ ─ ─ ─ ─ ┘
                     │
                     ▼
┌─────────────────────────────────────────────┐
│            set first target position        │
└─────────────────────────────────────────────┘
                     │
                     ▼
┌─────────────────────────────────────────────┐
│          set first FFL-trajectory-offset    │◄──────────┐
└─────────────────────────────────────────────┘           │
                     │                                     │
                     ▼                                     │
┌─────────────────────────────────────────────┐           │
│            calibration measurement           │◄────┐     │
│               (run trajectory)               │     │     │
└─────────────────────────────────────────────┘     │     │
                     │                               │     │
                     ▼                               │     │
                  ╱further╲       yes   ┌────────────────┐ │
                ╱ FFL-trajectory- ╲─────►│  set further   │─┘
                ╲   offset?      ╱       │FFL-trajectory- │
                  ╲           ╱          │    offset      │
                     │no                 └────────────────┘
                     ▼
┌ ─ ─ ─ ─ ─ ─ ─ ─ ─ ─ ─ ─ ─ ─ ─ ─ ─ ─ ─ ─ ─ ─ ┐
│          background measurement             │
└ ─ ─ ─ ─ ─ ─ ─ ─ ─ ─ ─ ─ ─ ─ ─ ─ ─ ─ ─ ─ ─ ─ ┘
                     │
                     ▼
                  ╱further╲       yes   ┌────────────────┐
                ╱  target   ╲───────────►│  set further   │
                ╲ position? ╱            │ target position│
                  ╲       ╱              └────────────────┘
                     │no
                     ▼
┌─────────────────────────────────────────────┐
│            end of data acquisition          │
└─────────────────────────────────────────────┘
```

Fig. 4

determine
- calibration volume
- MPI imaging sequence with sequence parameters
- magnetic fields to be generated by MPI scanner
- Tracer and/or environmental parameters

Emulate calibration volume by Magnetic Particle Spectrometer

calibration measurements
(applying determined magnetic fields)

**Multidimensional sinogram-based system matrix** $A_{Sinogram}$

Fig. 5

determine
- Target positions
- parameters
- calibration volume
- FFL trajectory offsets

↓

Discretize parameters

↓

calibration measurements
(applying magnetic fields by MPI scanner)

↓

individual acquisition system matrices

↓

Combination of individual acquisition system
matrices
➔ **generic system matrix**

↓

Pick best matching calibration data

↓

Concatenate picked calibration data

⇓

**Multidimensional sinogram-based
system matrix**

**Fig. 6**

Fig. 7

Fig. 8

Fig. 9

a)

b)

c)

Fig. 10

Fig. 11

**EUROPEAN SEARCH REPORT**

Application Number

EP 24 21 4298

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | US 2015/221103 A1 (KNOPP TOBIAS [DE]) 6 August 2015 (2015-08-06) * the whole document * | 1-14 | INV. G01R33/12 A61B5/0515 |
| A | VLADYSLAV GAPYAK ET AL: "Reconstruction Formulae for 3D Field-Free Line Magnetic Particle Imaging", ARXIV.ORG, CORNELL UNIVERSITY LIBRARY, 201 OLIN LIBRARY CORNELL UNIVERSITY ITHACA, NY 14853, 12 September 2023 (2023-09-12), XP091611260, * page 2 * | 1-14 | |
| A | WO 2024/042614 A1 (MITSUBISHI ELECTRIC CORP [JP]) 29 February 2024 (2024-02-29) * figures 1-6 * | 1-14 | |

TECHNICAL FIELDS SEARCHED (IPC)

G01R
A61B

The present search report has been drawn up for all claims

| Place of search | Date of completion of the search | Examiner |
|---|---|---|
| Munich | 9 April 2025 | Philipp, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 21 4298

This annex lists the patent family members relating to the patent documents cited in the above-mentioned European search report.
The members are as contained in the European Patent Office EDP file on
The European Patent Office is in no way liable for these particulars which are merely given for the purpose of information.

09-04-2025

| Patent document cited in search report | Publication date | Patent family member(s) | Publication date |
|---|---|---|---|
| US 2015221103 A1 | 06-08-2015 | DE 102012211662 A1<br>EP 2869760 A1<br>US 2015221103 A1<br>WO 2014005658 A1 | 09-01-2014<br>13-05-2015<br>06-08-2015<br>09-01-2014 |
| WO 2024042614 A1 | 29-02-2024 | CN 119677458 A<br>JP 7520261 B1<br>JP WO2024042614 A1<br>WO 2024042614 A1 | 21-03-2025<br>22-07-2024<br>29-02-2024<br>29-02-2024 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2015221103 A
- US 20170020407 A
- CN 115797493
- US 009651637 B
- US 20170020407 A1
- US 009651637 B2

**Non-patent literature cited in the description**

- **WEIZENECKER et al.** Magnetic particle imaging using a field free line. *Journal of Physics D: Applied Physics*, 2008, vol. 41 (10), 105009
- **WEIZENECKER et al.** Three-dimensional real-time in vivo magnetic particle imaging. *Physics in Medicine & Biology*, 2009, vol. 54 (5), L1
- **KAETHNER et al.** Axially Elongated Field-Free Point Data Acquisition in Magnetic Particle Imaging. *IEEE Transactions on Medical Imaging*, February 2015, vol. 34 (2), 381-387
- **VON GLADISS, A. et al.** Hybrid system calibration for multidimensional magnetic particle imaging.. *Physics in Medicine & Biology*, 2017, vol. 62 (9), 3392
- System Calibration Unit for Magnetic Particle Imaging: Focus Field Based System Function. **HALKOLA et al.** Magnetic Particle Imaging. Springer Proceedings in Physics. Springer, vol. 140
- **LI et al.** Fast System Matrix Generation Based on Single Angle Calibration in Open-Sided Field Free Line Magnetic Particle Imaging. *IEEE TRANSACTIONS ON BIOMEDICAL ENGINEERING*, April 2024, vol. 71 (4), 1209-1218
- **MOHN et al.** System Matrix Based Reconstruction for Pulsed Sequences in Magnetic Particle Imaging. *IEEE TRANSACTIONS ON MEDICAL IMAGING*, July 2022, vol. 41 (7), 1862-1873